# FASCICULE DE BREVET EUROPEEN

(11) **EP 2 102 543 B1**
(45) Date de publication et mention de la délivrance du brevet: **30.06.2010**
(21) Numéro de dépôt: 08701521.0
(22) Date de dépôt: 16.01.2008
(51) Int. Cl.: F16M 13/02, F16C 11/10, A61M 5/14

(54) **Dispositif de fixation muni de moyens de verrouillage**
Befestigungsvorrichtung mit einer Verriegelung
Fixation device provided with locking means

(30) Priorité: 20.01.2007 FR 0700393
(43) Date de publication de la demande: 23.09.2009
(73) Titulaire: Fresenius Vial SAS, 38590 Brézins (FR)
(72) Inventeur: PONÇON, Gilbert, F-38340 Pommiers La Placette (FR); TRAVERSAZ, Philippe, F-38140 Saint-blaise Du Buis (FR)
(74) Mandataire: Vièl, Frédérique
(86) Numéro de dépôt international: PCT/EP2008/050454
(87) Numéro de publication internationale: WO 2008/090068

(56) Documents cités:
- WO-A-01/36027
- FR-A- 2 881 507
- US-A- 2 692 153
- US-A- 5 014 956
- US-A- 5 829 723

## Description

L'invention concerne un dispositif pour fixer un objet sur un support, lequel dispositif comprend ledit objet à fixer et est muni de moyens de fixation comprenant une platine fixée sur l'une des parois de l'objet à fixer, de préférence la paroi arrière, et de moyens de serrage pour serrer les moyens de fixation sur le support, les moyens de serrage pouvant pivoter par rapport à la platine autour d'un axe (N) de sorte à pouvoir être rabattus contre la platine ou redressés perpendiculairement à ladite platine, des moyens de verrouillage étant prévus pour verrouiller les moyens de serrage dans la position d'utilisation redressée perpendiculairement à la platine.

Le dispositif de fixation de l'invention est destiné particulièrement à un objet devant être fixé dans une position spatiale privilégiée quelle que soit l'orientation du support. C'est le cas par exemple d'un module médical tel qu'un pousse seringue qui peut être fixé sur un mât vertical, sur un rail horizontal ou dans un rack. Quel que soit le support sur lequel il doit être fixé, il faut que son orientation dans l'espace soit toujours la même, c'est-à-dire que le haut du pousse seringue doit être en haut et le bas en bas. Il n'est en général pas possible d'utiliser ces pompes dans une position « couchée », c'est-à-dire pivotée de 90° par rapport à un axe horizontal.

On connaît de FR 2 881 507 A1 un dispositif conforme au préambule. Le module médical est muni sur sa face arrière de moyens de fixation pouvant pivoter entre deux positions privilégiées pour permettre sa fixation aussi bien sur un support horizontal que sur un support vertical. Ces moyens de fixation sont constitués d'une part d'une platine fixée à la face arrière du module de sorte à pouvoir pivoter entre deux positions perpendiculaires, et d'autre part de moyens de serrage, en l'occurrence une noix de serrage munie d'un mors et d'une vis de serrage, pouvant pivoter pour être au choix rabattus contre la platine, notamment pour le rangement, ou au contraire redressés dans une position perpendiculaire à ladite platine pour permettre la fixation du module sur le support choisi. Des moyens de verrouillage sont prévus pour empêcher les moyens de serrage de se rabattre tout seuls lorsqu'ils sont en position redressée. Ces moyens de verrouillage comprennent un verrou pivotant autour d'un axe de pivotement confondu avec l'axe de pivotement de la platine et ayant une partie excentrique avec un bord plat qui, quand le verrou est actionné, prend appui contre la face intérieure des moyens de serrage et en bloque le pivotement. Lorsque le verrou est déverrouillé, les moyens de serrage peuvent se rabattre sur celui-ci. De plus, pour éviter que les moyens de serrage ne se redressent de façon inopinée, on a prévu sur les moyens de serrage et/ou sur la platine des moyens de retenue pour retenir les moyens de serrage contre la platine, sans pour autant les bloquer, lorsque les moyens de serrage sont en position rabattue contre ladite platine. Ces moyens de retenue sont de préférence constitués par des aimants.

Ce dispositif permet une grande souplesse d'utilisation en autorisant une fixation sur un support horizontal ou sur un support vertical, tout en permettant à la noix de fixation de se rabattre contre le module lors du rangement. Les aimants évitent que la noix ne se redresse lors de son stockage tandis que les moyens de verrouillage empêchent la noix de se rabattre alors que le module est fixé à son support.

Cependant, l'opération de verrouillage n'est pas toujours facile. En effet, il faut d'une part maintenir la noix en position redressée perpendiculairement à la platine, et d'autre part faire pivoter d'un quart de tour le verrou pour bloquer la noix dans cette position redressée. Soit le verrou peut pivoter facilement, auquel cas la noix n'est pas fermement bloquée en position redressée laissant au module un certain jeu lorsqu'il est fixé sur son support, soit au contraire le verrou pivote difficilement, ce qui certes assure un bon blocage de la noix en position redressée, mais nécessite une certaine force pour assurer un bon verrouillage. Dans ce cas, il n'est pas rare que le personnel renonce à verrouiller correctement la noix dans sa position redressée. Or, si le verrou n'est pas entièrement tourné, la noix n'est pas correctement bloquée et risque de se rabattre lors de l'utilisation, en entraînant avec elle le module. De plus, le verrou se trouve dans l'angle formé par la platine et la noix, autrement dit dans un endroit difficile d'accès.

L'objectif de l'invention est de proposer un dispositif de fixation selon le préambule qui permette de verrouiller facilement les moyens de serrage dans leur position redressée. Un autre objectif de l'invention est de concevoir le dispositif de fixation de telle sorte qu'il soit possible de renoncer aux moyens de retenue distincts, notamment sous la forme d'aimants.

L'objectif principal est atteint conformément à l'invention par le fait que les moyens de verrouillage sont constitués par un doigt pouvant être déplacé entre une première position rentrée dans laquelle, lorsque les moyens de serrage sont en position redressée, il est placé par une surface d'appui contre la face interne desdits moyens de serrage en les bloquant dans cette position et une deuxième position écartée dans laquelle il permet aux moyens de serrage de pivoter librement en direction rabattue ou en position redressée en passant devant le doigt écarté de sorte qu'en position rabattue des moyens de serrage, le doigt se trouve face ou contre une des faces des moyens de serrage autre que la face intérieure. Ainsi, il suffit d'écarter le doigt pour permettre aux moyens de serrage de pivoter dans un sens ou dans l'autre, alors qu'en position rentrée, il bloque ces moyens de serrage s'ils sont en position redressée. Il n'est plus nécessaire de faire pivoter un verrou placé dans l'angle formé par les moyens de serrage et la platine.

Dans un mode de réalisation particulière de l'invention, il est prévu un ressort qui tend à maintenir le doigt dans la position rentrée. Autrement dit, dès que les moyens de serrage sont redressés, le doigt prend automatiquement sa position de blocage. De plus, même si le module ou son support sont soumis à des secousses, le doigt reste en position rentrée et le module ne risque pas de se rabattre intempestivement.

Pour permettre un guidage automatique du doigt lors du redressement des moyens de serrage sans être obligé de l'écarter manuellement, il est préférable de munir le bord extérieur des moyens de serrage d'une surface inclinée contre laquelle peut glisser le doigt lors du pivotement des moyens de serrage entre leur position rabattue et leur position redressée. Le doigt, durant ce mouvement de pivotement, contourne le bord extérieur des moyens de serrage contre l'effet du ressort jusqu'à ce qu'il dépasse le bord extérieur et qu'il aille bloquer les moyens de serrage dans leur position redressée.

Cette surface inclinée sert de came pour le doigt. Celui-ci est soumis à la force du ressort qui tend, quelle que soit sa position, à le ramener dans sa position rentrée. Lorsque les moyens de serrage sont rabattus, le doigt, sous l'effet du ressort, appuie sur la surface inclinée. Plus les moyens de serrage sont écartés de la position rabattue en direction de la position redressée, plus la surface inclinée force le doigt à s'écarter de sa position rentrée jusqu'à ce qu'il soit suffisamment écarté pour laisser passer le bord extérieur des moyens de serrage. Dès que ce bord extérieur a dépassé le doigt, celui-ci quitte la surface inclinée et retourne automatiquement dans sa position rentrée sous l'effet du ressort.

Cette solution a pour deuxième avantage de servir de moyens de retenue. En effet, le doigt en prenant appui sur la surface inclinée tend à plaquer les moyens de serrage dans leur position rabattue. Les moyens de serrage ne peuvent être redressés qu'en surpassant l'effet de rappel du ressort. Celui-ci est dimensionné de telle sorte qu'il soit suffisamment fort pour retenir les moyens de serrage en position rabattue même contre l'effet de la gravité, mais pas de trop pour que l'utilisateur puisse redresser les moyens de serrage sans effort excessif.

Pour déverrouiller le doigt lorsqu'il est en position de verrouillage des moyens de serrage, il est préférable de le munir d'un bouton de déverrouillage sur lequel il est possible d'appuyer ou de tirer pour déplacer le doigt dans sa position écartée, le cas échéant contre l'effet du ressort.

Le plus simple consiste à placer le doigt sur l'axe de rotation des moyens de serrage. Le doigt peut être par exemple placé à l'extrémité d'une tige servant d'axe pour la rotation des moyens de serrage. De même, il est préférable que le ressort soit un ressort de compression, de préférence placé à l'intérieur de l'axe de rotation des moyens de serrage. Il est préférable de concevoir le doigt de telle sorte qu'il puisse être déplacé entre ses deux positions extrêmes selon un mouvement de translation.

Conformément à un mode de réalisation particulier de l'invention, les moyens de serrage sont munis de moyens d'actionnement disposés sur lesdits moyens de serrage de sorte à être manipulables depuis le côté latéral des moyens de fixation. Ainsi, l'opérateur peut accéder aux moyens d'actionnement depuis le côté latéral des moyens de fixation, donc du module, ce qui est beaucoup plus commode que de devoir y accéder de l'arrière, comme c'est souvent le cas dans l'état de la technique.

Pour permettre une fixation aussi bien horizontale que verticale du module, il est préférable que la platine puisse pivoter de 90° autour d'un axe (P).

Il est préférable que les moyens de serrage comprennent une noix munie d'un mors et d'une vis de serrage pouvant tous les deux pivoter autour de l'axe (N). C'est un mode de réalisation particulièrement simple et pratique, facile à réaliser industriellement.

Pour faciliter l'utilisation des moyens de fixation selon l'invention, il est préférable de prévoir des moyens d'arrêt pour limiter le mouvement de pivot des moyens de fixation pivotants, de préférence entre deux positions extrêmes situées à 90° l'une de l'autre. Ainsi, les moyens de fixation ne peuvent prendre que deux positions perpendiculaires l'une à l'autre. Il serait cependant également possible de permettre aux moyens de fixation pivotants de prendre quatre positions distinctes décalées de 90° les unes par rapport aux autres, présentant ainsi deux positions possibles pour chaque type de support horizontal ou vertical. Les moyens d'arrêt peuvent consister par exemple en un picot de guidage et d'arrêt solidaire de la platine et coopérant avec une gorge de guidage réalisée dans la paroi de l'objet à fixer ou inversement.

Il peut être préférable de ne permettre aux moyens de serrage de se redresser que lorsque la platine est en position d'utilisation, autrement dit d'interdire aux moyens de serrage de se redresser dans une position intermédiaire entre deux positions d'utilisation. Pour cela, il est possible de prévoir des moyens de guidage pour guider le mouvement de pivot des moyens de serrage dans l'une ou l'autre position d'utilisation. Cela peut être réalisé par exemple en concevant ces moyens de guidage sous la forme d'une nervure de guidage réalisée sur les moyens de serrage et d'au moins une rainure de guidage réalisée dans la paroi de l'objet à fixer face à l'une au moins des positions d'utilisation et dans laquelle peut pénétrer la nervure de guidage lorsque les moyens de serrage pivotent autour de l'axe (N) en direction de la position d'utilisation. Si la platine n'est pas dans l'une des positions d'utilisation, la nervure ne pourra pas pénétrer dans l'une des gorges de guidage. Elle bloquera alors le mouvement de pivot des moyens de serrage en direction de la position redressée. Pour redresser les moyens de serrage en position d'utilisation, il faut donc que la platine soit correctement positionnée.

Afin que le dispositif de fixation soit le plus stable possible en position ouverte et que l'objet à fixer ne risque pas de pivoter sur le côté, il est préférable de prévoir un indexage angulaire en fin de redressement des moyens de serrage. Pour cela, des moyens de blocage sont prévus pour bloquer la platine dans au moins l'une de ses positions d'utilisation, ces moyens de blocage comprenant de préférence un index placé sur les moyens de serrage qui pénètre, lorsque les moyens de serrage sont redressés en position d'utilisation, dans une gorge d'indexage réalisée dans la paroi de l'objet à fixer. Alors que les moyens de guidage n'avaient en principe pas besoin de permettre un mouvement sans jeu, il est préférable que cet indexage angulaire soit le plus précis possible et sans jeu.

Il est préférable que les moyens de fixation comprennent une rainure dont les dimensions correspondent aux dimensions habituelles des supports horizontaux, ladite rainure étant réalisée de préférence dans le mors des moyens de serrage. Il est ainsi possible de placer le module sur un rail horizontal de section rectangulaire sans qu'il risque de pivoter sous l'effet de son propre poids avant que les moyens de serrage ne soient mis en place. Il est possible de prévoir plusieurs dimensions de rail en imbriquant les unes dans les autres plusieurs rainures, de la plus large vers la plus fine.

Dans une variante de réalisation de l'invention, les moyens de fixation sont munis de moyens pour faire pénétrer l'objet à fixer dans un emplacement, par exemple dans un rack, lesdits moyens d'introduction comprenant de préférence un picot parallèle à la paroi arrière de l'objet et pouvant décrire un mouvement circulaire par rapport à la paroi arrière de l'objet à fixer, ledit picot pouvant pénétrer dans une gorge de guidage réalisée dans une des parois latérales de l'emplacement du rack, de sorte que le mouvement du picot dans la gorge de guidage provoque l'entrée de l'objet à fixer dans l'emplacement. En faisant pivoter la platine sur son axe (P), le picot pénètre dans la gorge de guidage. Plus il pénètre dans celle-ci, plus la gorge le contraint à avancer vers le fond de l'emplacement en entraînant le module avec lui. Il n'est donc pas nécessaire d'appuyer sur le module pour le faire pénétrer dans l'emplacement, au risque de renverser tout le rack.

Il peut être favorable de prévoir un capteur pour détecter le bon positionnement de l'objet à fixer dans l'emplacement, le capteur étant de préférence un capteur à effet Hall coopérant avec un aimant placé dans les moyens de fixation pivotants, de préférence à proximité du picot. Le capteur peut être positionné par exemple près de la gorge de guidage, de préférence à proximité de la section terminale de ladite gorge.

Dans un mode de réalisation particulier de l'invention, le dispositif de fixation reçoit des moyens de verrouillage caractérisés par un doigt muni d'une surface d'appui dimensionnée pour pouvoir coopérer avec la surface intérieure de moyens de serrage, ainsi que le cas échéant par une tige pouvant servir d'axe de rotation (N) pour les moyens de serrage et/ou par un bouton de déverrouillage pouvant être fixé notamment à l'autre extrémité de la tige et/ou par un ressort.

Un exemple de réalisation de l'invention est présenté ci-dessous en comparaison avec un dispositif de l'état de la technique. Les figures montrent :
- Figure 1 :: un dispositif de fixation de l'art antérieur dans une position permettant la fixation sur un support vertical ;
- Figure 2 :: le dispositif de la figure 1 dans une position permettant une fixation sur un support horizontal ;
- Figure 3 :: le dispositif amélioré conforme à l'invention en position redressée permettant la fixation sur un support vertical, les moyens de verrouillage étant en position rentrée ;
- Figure 4 :: la platine et les moyens de serrage du dispositif de la figure 3 avec les moyens de serrage en position intermédiaire et les moyens de verrouillage en position écartée ;
- Figure 5 :: le dispositif de la figure 3 avec les moyens de serrage en position rabattue et les moyens de verrouillage en position rentrée ;

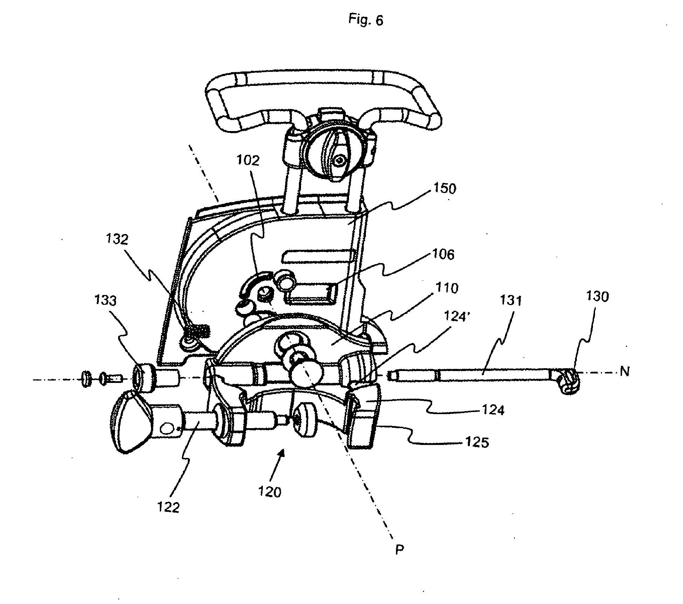

- Figure 6 :: le dispositif de la figure 3 en vue éclatée ;
- Figures 7a-7c: des vues schématiques de côté du dispositif de la figure 3 avec les moyens de serrage (a) en position redressée, (b) en position intermédiaire et (c) en position rabattue.

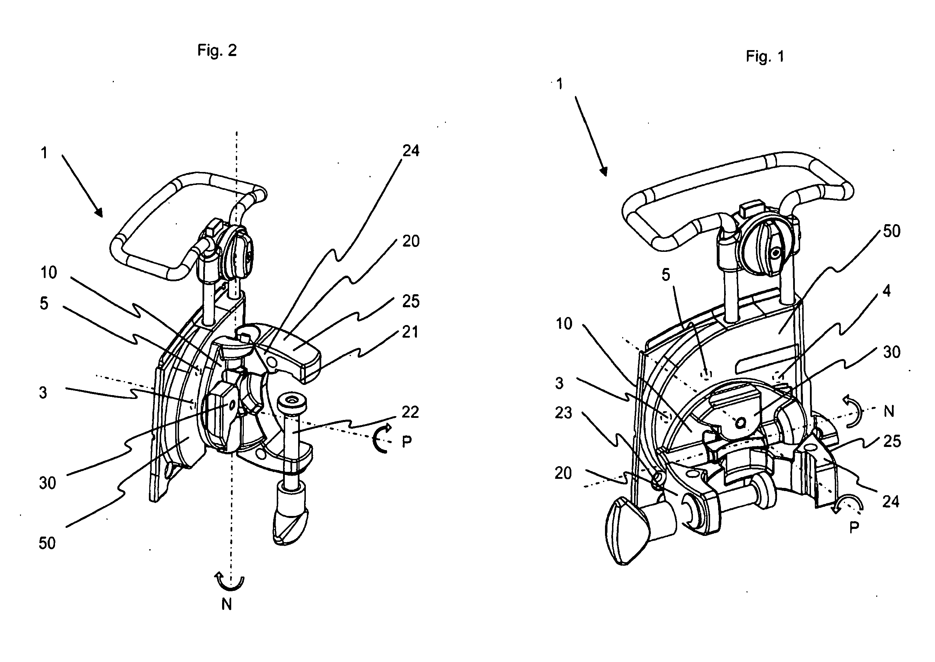

Pour plus de clarté, les figures 1 et 2 présentent un dispositif de fixation (1) de l'art antérieur tel qu'il est connu du document FR 2 881 507 A1. Le dispositif de fixation de l'invention (100) est une amélioration de ce dispositif connu (1). Ces deux dispositifs (1, 100), se composent essentiellement de trois parties : d'une platine pivotante (10, 110), d'une noix pivotante (20, 120) et d'un verrou (30, 130). La platine pivotante (10, 110) et la noix pivotante (20, 120) constituent les moyens de fixation, la noix pivotante (20, 120) constituant les moyens de serrage et le verrou (30, 130) les moyens de verrouillage.

La platine pivotante (10, 110) est fixée sur la paroi arrière (50, 150) de l'objet à fixer. Elle peut pivoter de 90° autour de son axe (P).

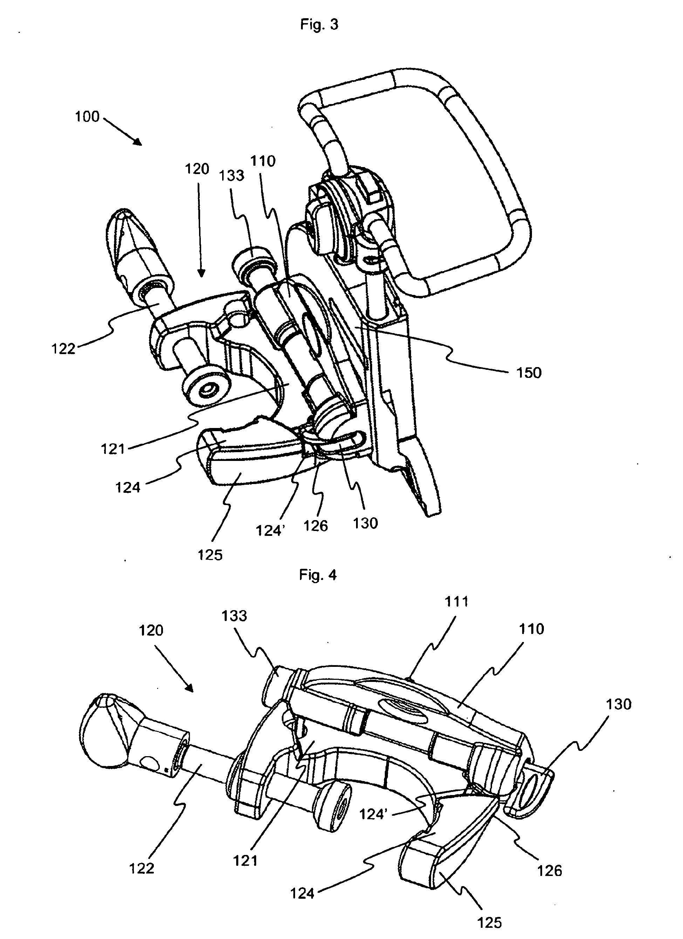

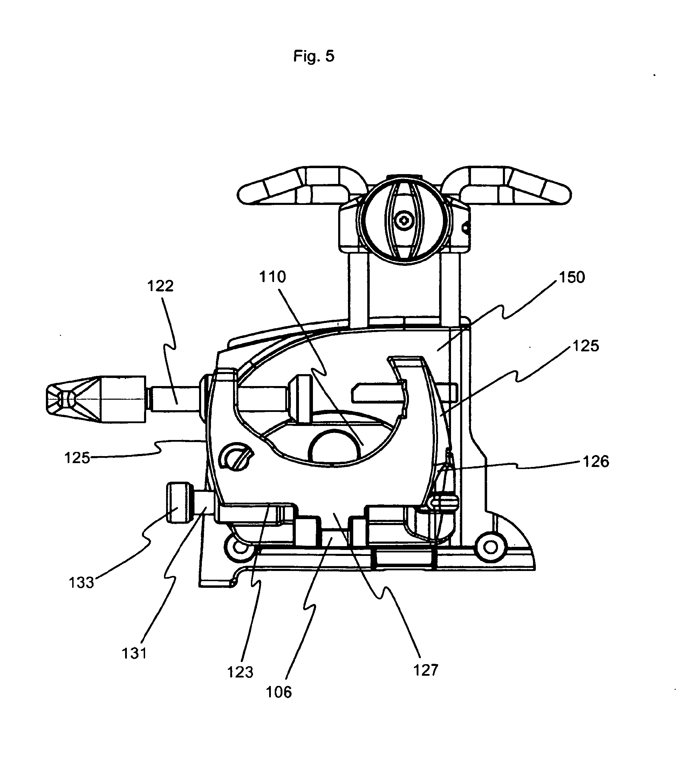

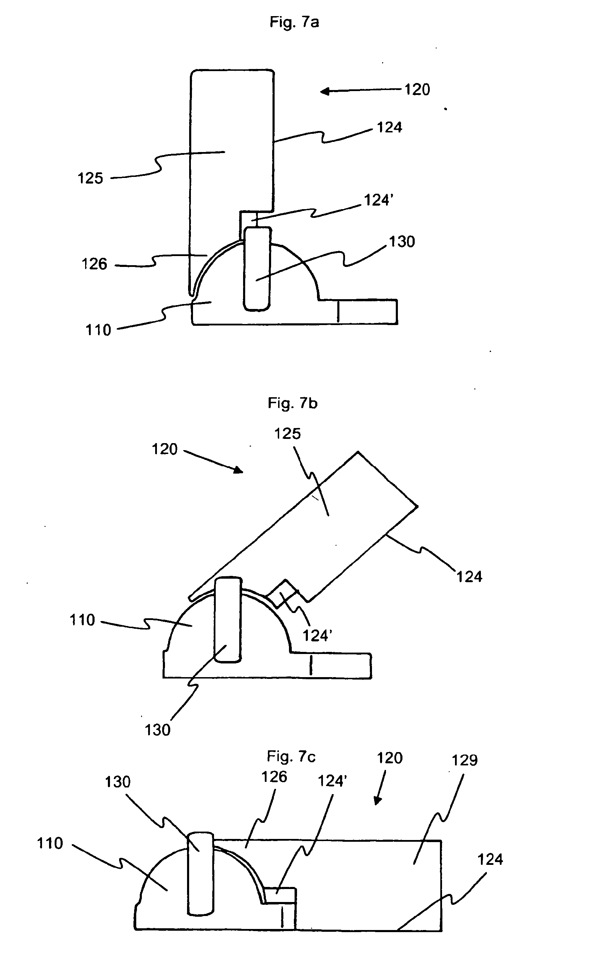

La noix pivotante (20, 120) est constituée essentiellement d'un mors (21, 121) et d'une vis de serrage (22, 122). Elle est fixée sur la platine (10, 110) de sorte à pouvoir pivoter autour de son axe (N) entre une position rabattue et une position redressée après avoir réalisé un quart de tour autour de l'axe (N). Les figures 1 à 3 et 7a montrent les moyens de serrage (20, 120) dans la position redressée, les figures 4 et 7b dans une position intermédiaire et les figures 5 et 7c en position rabattue.

Les moyens de serrage (20, 120) présentent une face latérale intérieure (24, 124) qui lorsqu'elle est rabattue fait face à la platine (10, 110), une face latérale extérieure opposée à la face latérale intérieure, deux bords extérieurs (25, 125) et une face arrière (23, 123), face faisant face à la platine lorsque les moyens de serrage (20, 120) sont en position redressée. Cette face arrière (23, 123) peut servir de butée pour empêcher les moyens de serrage de dépasser un angle de 90° en venant prendre appui sur la paroi arrière (50, 150) de l'objet à fixer. D'autres types de butées sont bien sûr envisageables.

La vis de serrage (22, 122) sert de moyens d'actionnement des moyens de serrage. Comme le montrent les figures, elle est toujours accessible depuis le côté latéral des moyens de fixation, c'est-à-dire le côté latéral du module sur lequel ils sont fixés, et ce que les moyens de fixation soient redressés ou rabattus et qu'ils soient en position horizontale ou en position verticale. La main de l'utilisateur ne doit pas chercher derrière le module une vis de serrage redressée perpendiculairement à la face arrière de ce module.

Pour des raisons de sécurité, il faut verrouiller les moyens de serrage (20, 120) dans leur position ouverte à l'aide de moyens de verrouillage. Conformément à l'invention, ces moyens de verrouillage sont constitués par un doigt (130) pouvant être déplacé entre une première position rentrée (figures 3 et 5) et une deuxième position écartée (figure 4).

Dans la position rentrée de la figure 3, si les moyens de serrage sont en position redressée, le doigt se trouve contre la face interne (124) des moyens de serrage (120) en les bloquant dans cette position. Les moyens de serrage (120) se trouvent donc bloqués contre tout mouvement de pivot d'une part par le doigt (130) qui les empêche de retourner dans la position rabattue et d'autre part par leur face arrière (123) qui sert de butée, les empêchant de dépasser cette position redressée à 90°.

Dans la position écartée de la figure 4, le doigt permet aux moyens de serrage (120) de pivoter librement en direction de la platine (120) en passant devant le doigt (130) écarté. En position rabattue des moyens de serrage (120), le doigt (130) se trouve face non plus à la face intérieure (124) mais au bord extérieur (125) comme le montre les figures 5 et 7c. Il serait également possible qu'il se trouve contre la face extérieure latérale de la noix après avoir dépassé le bord extérieur (125).

Pour simplifier les choses, le doigt (130) est placé à l'extrémité d'une tige (131) qui sert également d'axe (N) pour le pivot des moyens de serrage (120). Par conséquent, la platine (110) et les moyens de serrage (120) sont articulés ensemble par cette tige (131).

Pour assurer un maintien du doigt (130) dans sa position rentrée lorsque les moyens de serrage (120) sont redressés, on a prévu un ressort (132) qui tend à rappeler le doigt (130) dans cette position de sécurité. Par conséquent, pour rabattre les moyens de serrage (120) lorsqu'ils sont verrouillés en position redressée, il faut appuyer sur un bouton de déverrouillage (133) situé sur la tige (131) à l'opposé du doigt (130).

Pour commander automatiquement le mouvement du doigt (130), les moyens de serrage (120) sont munis d'une surface inclinée (126) placée sur leur bord extérieur (125). Cette surface inclinée (126) s'étend de la face latérale extérieure vers la face latérale intérieure (124) des moyens de serrage, en formant sensiblement un arc de cercle. L'inclinaison de cette surface inclinée (126) est telle que son extrémité inférieure située du côté de la face extérieure est plus proche du plan médian des moyens de serrage (120), plan perpendiculaire à l'axe de pivotement (N) de ces moyens de serrage, que son extrémité supérieure située du côté de sa face intérieure (124).

Afin d'assurer un verrouillage efficace et sans jeu des moyens de serrage (120) en position redressée, la face intérieure (124) est munie elle aussi d'une surface inclinée (124') contre laquelle glisse le doigt (130) dès qu'il a quitté l'extrémité supérieure de la première surface inclinée (126). Cette deuxième surface inclinée (124') ne s'écarte angulairement que peu de l'axe (N) de sorte qu'il n'est pas possible d'écarter le doigt (130) en tentant de rabattre les moyens de serrage (120) en direction de la platine (110). Cette seconde surface inclinée garantit que le doigt (130) appuie effectivement, c'est-à-dire sans jeu, contre la face intérieure (124) des moyens de serrage (120). Le doigt, sous l'effet du ressort (132), glisse sur cette seconde surface inclinée (124') en forçant les moyens de serrage à finir leur mouvement de redressement jusqu'à ce que ceux-ci soient sensiblement perpendiculaires à la platine (110). Ces deux surfaces inclinées (124', 126) sont bien visibles sur les figures 7a à 7c.

On voit donc que lorsque les moyens de serrage (120) sont rabattus contre la platine (110), le doigt (130) appuie contre l'extrémité inférieure de la surface inclinée (126), à proximité de la face latérale extérieure de la noix (120). Le fait de redresser les moyens de serrage (120) provoque le glissement du doigt (130) sur la surface inclinée (126) en l'écartant de sa position rentrée contre l'effet de rappel du ressort (132). Cet effet de rappel du ressort (132) est suffisant pour, en l'absence de sollicitation suffisante, maintenir en position rabattue les moyens de serrage (120) quelle que soit leur position. Il n'est donc plus nécessaire d'utiliser des aimants comme dans le dispositif de l'état de la technique.

Par contre, si l'utilisateur surpasse la force de rappel du ressort (132), il peut redresser les moyens de serrage (120) jusqu'à ce qu'ils atteignent la position redressée perpendiculaire à la platine (110). Durant tout ce mouvement, le doigt (130) glisse sur la surface inclinée (126) en s'écartant de plus en plus de sa position rentrée (voir figures 7a à 7c). Dès que la position redressée est atteinte, le doigt (130) dépasse l'extrémité supérieure de la surface inclinée (126). N'ayant plus de soutien, il retombe sous l'effet de rappel du ressort (132) dans sa position de repos, la position rentrée, en glissant contre la deuxième surface inclinée (124') beaucoup plus pentue que la première. Les moyens de serrage (120) sont ainsi verrouillés dans leur position de travail.

Pour rabattre les moyens de serrage (120), il suffit à l'utilisateur d'appuyer sur le bouton de déverrouillage (133). Le doigt (130) est alors suffisamment écarté de sa position de repos pour laisser pivoter librement les moyens de serrage (120) autour de l'axe (N) en passant devant le doigt (130) écarté.

Comme dans l'art antérieur, les moyens de serrage (120) peuvent en position ouverte prendre deux positions distinctes décalées de 90° l'une par rapport à l'autre. L'objet à fixer peut donc être fixé sur un mât vertical lorsque les moyens de serrage (120) sont redressés en position horizontale (figure 1) ou sur un rail horizontal lorsqu'ils sont redressés en position verticale (figure 2). Les mêmes moyens de fixation permettent donc de fixer l'objet dans une position spatiale privilégiée.

### Liste des références :

| Art antérieur (Fig. 1 et 2) | Invention (Fig. 3 à 7) | |
|---|---|---|
| 1 | 100 | Dispositif de fixation |
| | 102 | Gorge de pivot pour la platine |
| | 106 | Rainure de guidage |
| | | |
| 10 | 110 | Platine pivotante |
| | 111 | Picot de guidage et d'arrêt |
| | | |
| 20 | 120 | Noix pivotante |
| 21 | 121 | Mors |
| 22 | 122 | Vis de serrage |
| 23 | 123 | Face arrière de la noix |
| 24 | 124 | Face latérale intérieure de la noix |
| 25 | 125 | Bord extérieur |
| | 126 | Surface inclinée |
| | 127 | Nervure de guidage |
| | | |
| 30 | 130 | Verrou / Doigt |
| | 131 | Tige |
| | 132 | Ressort |
| | 133 | Bouton de déverrouillage |
| | | |
| 50 | 150 | Paroi arrière de l'objet à fixer |

## Revendications

1. Dispositif (100) pour fixer un objet sur un support, comprenant ledit objet à fixer et muni de moyens de fixation comprenant une platine (110) fixée sur l'une des parois de l'objet à fixer, de préférence la paroi arrière, et des moyens de serrage (120) pour serrer les moyens de fixation sur le support, les moyens de serrage (120) pouvant pivoter par rapport à la platine (110) autour d'un axe (N) de sorte à pouvoir être rabattus contre la platine (110) ou redressés perpendiculairement à ladite platine (110), des moyens de verrouillage étant prévus pour verrouiller les moyens de serrage (120) dans la position d'utilisation perpendiculaire à la platine (110), **caractérisé en ce que** les moyens de verrouillage sont constitués par un doigt (130) pouvant être déplacé entre une première position rentrée dans laquelle, lorsque les moyens de serrage (120) sont en position redressée, il est placé par une surface d'appui contre la face interne (124) desdits moyens de serrage (120) en les bloquant dans cette position et une deuxième position écartée dans laquelle il permet aux moyens de serrage (120) de pivoter librement en direction de la platine (110) en passant devant le doigt (130) écarté de sorte qu'en position rabattue des moyens de serrage (120), le doigt (130) se trouve face ou contre une des faces (125) des moyens de serrage (120) autre que la face intérieure (124).

2. Dispositif (100) selon la revendication 1, **caractérisé en ce qu'**il est prévu un ressort (132) qui tend à maintenir le doigt (130) dans la position rentrée.

3. Dispositif (100) selon la revendication 2, **caractérisé en ce que** le bord extérieur (125) des moyens de serrage (120) est muni d'une surface inclinée (126) contre laquelle peut glisser le doigt (130) lors du pivotement des moyens de serrage (120) entre leur position rabattue et leur position redressée, le doigt (130), durant ce mouvement de pivotement, contournant le bord extérieur (125) des moyens de serrage (120) contre l'effet du ressort (132) jusqu'à ce qu'il dépasse le bord extérieur (125) et qu'il aille bloquer les moyens de serrage (120) dans leur position redressée.

4. Dispositif (100) selon l'une des revendications précédentes, **caractérisé en ce que** le doigt (130) est muni d'un bouton de déverrouillage (133) sur lequel il est possible d'appuyer ou de tirer pour déplacer le doigt (130) dans sa position écartée, le cas échéant contre l'effet du ressort (132).

5. Dispositif (100) selon l'une des revendications précédentes, **caractérisé en ce que** le doigt (130) est placé sur l'axe de rotation (N) des moyens de serrage (120).

6. Dispositif (100) selon l'une des revendications 2 à 3, **caractérisé en ce que** le ressort (132) est un ressort de compression de préférence placé à l'intérieur de l'axe de rotation (N) des moyens de serrage (120).

7. Dispositif (100) selon l'une des revendications précédentes, **caractérisé en ce que** le doigt (130) peut être déplacé entre ses deux positions extrêmes selon un mouvement de translation.

8. Dispositif (100) selon l'une des revendications précédentes, **caractérisé en ce que** les moyens de serrage (120) sont munis de moyens d'actionnement (122) disposés sur les moyens de serrage (120) de sorte à être manipulables depuis un côté latéral des moyens de fixation.

9. Dispositif (100) selon l'une des revendications précédentes, **caractérisé en ce que** les moyens de serrage comprennent une noix (120) munie d'un mors (121) et d'une vis de serrage (122) pouvant pivoter tous les deux autour de l'axe (N).

10. Dispositif (100) selon l'une des revendications précédentes, **caractérisé en ce que** la platine (110) peut pivoter de 90° autour d'un axe (P) en permettant indifféremment la fixation de l'objet sur un support vertical ou sur un support horizontal.

11. Dispositif (100) selon l'une des revendications précédentes, **caractérisé en ce que** des moyens d'arrêt sont prévus pour limiter le mouvement de pivot des moyens de fixation pivotants (110, 120), de préférence entre deux positions extrêmes situées à 90° l'une de l'autre, ces moyens d'arrêt comprenant de préférence un picot de guidage et d'arrêt (111) solidaire de la platine (110) coopérant avec une gorge de guidage (102) réalisée dans la paroi (150) de l'objet à fixer ou inversement.

12. Dispositif (100) selon l'une des revendications 10 ou 11, **caractérisé en ce que** des moyens de guidage sont prévus pour guider le mouvement de pivot des moyens de serrage (120) dans l'une ou l'autre position d'utilisation, ces moyens de guidage comprenant de préférence une nervure de guidage (127) réalisée sur les moyens de serrage (120) et d'au moins une rainure de guidage (106) réalisée dans la paroi (150) de l'objet à fixer face à l'une au moins des positions d'utilisation et dans laquelle peut pénétrer la nervure de guidage lorsque les moyens de serrage (120) pivotent autour de l'axe (N) en direction de la position d'utilisation.

13. Dispositif (100) selon l'une des revendications 10 à 12, **caractérisé en ce que** des moyens de blocage sont prévus pour bloquer la platine (110) dans au moins l'une de ses positions d'utilisation, ces moyens de blocage comprenant de préférence un index placé sur les moyens de serrage (120) qui pénètre, lorsque les moyens de serrage (120) sont redressés en position d'utilisation, dans une gorge d'indexage réalisée dans la paroi de l'objet à fixer.

14. Dispositif (100) selon l'une des revendications précédentes, **caractérisé en ce que** les moyens de serrage pivotants (120) comprennent une rainure dont les dimensions correspondent aux dimensions habituelles des supports horizontaux, ladite rainure étant réalisée de préférence dans le mors (121) des moyens de serrage (120).

15. Dispositif (100) selon l'une des revendications précédentes, **caractérisé en ce que** les moyens de fixation sont munis de moyens pour faire pénétrer l'objet à fixer sur un emplacement, par exemple dans un rack, lesdits moyens d'introduction comprenant de préférence un picot parallèle à la paroi arrière (150) de l'objet et pouvant décrire un mouvement circulaire par rapport à ladite paroi arrière (150), ledit picot pouvant pénétrer dans une gorge de guidage réalisée dans une paroi latérale de l'emplacement du rack, de sorte que le mouvement du picot dans la gorge de guidage provoque l'entrée de l'objet à fixer dans l'emplacement.

16. Dispositif (100) selon la revendication précédente, **caractérisé en ce qu'**un capteur est prévu pour détecter le bon positionnement de l'objet à fixer dans l'emplacement, le capteur étant de préférence un capteur à effet Hall coopérant avec un aimant placé dans les moyens de fixation, de préférence à proximité du picot.

17. Dispositif selon l'une des revendications 1 à 16, **caractérisé en ce que** les moyens de verrouillage sont munis d'un doigt (130) muni d'une surface d'appui dimensionnée pour pouvoir coopérer avec la surface intérieure de moyens de serrage (120), ainsi que par une tige (131) pouvant servir d'axe de rotation (N) pour les moyens de serrage (120) et/ou par un bouton de déverrouillage (133) pouvant être fixé notamment à l'autre extrémité de la tige (131) et/ou par un ressort (132).

## Claims

1. Device (100) for securing an object on a holder, which device comprises the said object to be fixed and is equipped with fixation means comprising a platen (110) fixed to one of the walls of the object to be secured, preferably the rear wall, and tightening means (120) for tightening the fixation means to the holder, the tightening means (120) being capable of pivoting relative to the platen (110) about an axis (N) so that they can be folded back against the platen or raised perpendicularly to said platen (110), locking means being provided to lock the tightening means (120) in the use position perpendicular to the platen (110), **characterized in that** the locking means are constituted by a tab (130) capable of displacement between a first stowed position in which, when the tightening means (120) are in the raised position, it is placed by a bearing surface against the inner face (124) of said tightening means (120) and blocks them in this position, and a second drawn out position in which it allows the tightening means (120) to pivot freely towards the platen (110) while passing in front of the drawn out tab (130) so that, in the folded position of the tightening means (120), the tab (130) faces or bears against one of the faces (125) of the tightening means (120) different from the inner face (124).

2. Device (100) according to claim 1, **characterized in that** a spring (132) is provided which tends to maintain the tab (130) in the stowed position.

3. Device (100) according to claim 2, **characterized in that** the outer edge (125) of the tightening means (120) is equipped with an inclined surface (126) against which the tab (130) can slide when the tightening means (120) pivot between their folded position and their raised position, wherein the tab (130), during this pivoting movement, passes around the outer edge (125) of the tightening means (120) against the force of the spring (132) until it reaches beyond from the outer edge (125) and goes on to block the tightening means (120) in their raised position.

4. Device (100) according to any one of the preceding claims, **characterized in that** the tab (130) is equipped with an unlock button (133) which can be pushed or pulled to move the tab (130) in its drawn out position, possibly against the force of the spring (132).

5. Device (100) according to any one of the preceding claims, **characterized in that** the tab (130) is placed on the rotation axis (N) of the tightening means (120).

6. Device (100) according to any one of claims 2 to 3, **characterized in that** the spring (132) is a compression spring, preferably placed inside the rotation axis (N) of the tightening means (120).

7. Device (100) according to any one of the preceding claims, **characterized in that** the tab (130) can be moved between its two extreme positions by a translation movement.

8. Device (100) according to any one of the preceding claims, **characterized in that** the tightening means (120) are equipped with actuating means (122) disposed on the tightening means (120) so that they can be manipulated from a lateral side of the fixation means.

9. Device (100) according to any one of the preceding claims, **characterized in that** the tightening means comprise a yoke (120) equipped with a jaw (121) and a tightening screw (122), both of which can pivot about the axis (N).

10. Device (100) according to any one of the preceding claims, **characterized in that** the platen (110) can pivot by 90 about an axis (P) while allowing securing the object indifferently to a vertical holder or to a horizontal holder.

11. Device (100) according to any one of the preceding claims, **characterized in that** stop means are provided to limit the pivoting movement of the pivotable fixation means (110, 120), preferably, between two extreme positions located at 90° from each other, these stop means comprising preferably a guiding and stop spike (111) integral with the platen (110) and which cooperates with a guiding groove (102) provided in the wall (150) of the object to be secured, or vice versa.

12. Device (100) according to any one of claims 10 or 11, **characterized in that** guiding means are provided for guiding the pivoting movement of the tightening means (120) into one or the other of the use positions, these guiding means comprising preferably a guiding rib (127) provided on the tightening means (120) and of at least one guiding groove (106) provided in the wall (150) of the object to be secured facing at least one of the use positions, and in which the guiding rib can be inserted when the tightening means (120) pivot about axis (N) toward the use position.

13. Device (100) according to any one of claims 10 to 12, **characterized in that** blocking means are provided to block the platen (110) in at least one of its use positions, these blocking means comprising preferably an index placed on the tightening means (120), which is inserted into an indexing groove provided in the wall of the object to be secured when the tightening means (120) are raised in use position.

14. Device (100) according to any one of the preceding claims, **characterized in that** the pivotable fixation means (120) comprise a groove whose dimensions correspond to the usual dimensions of the horizontal holders, said groove being preferably provided in the jaw (121) of the tightening means (120).

15. Device (100) according to any one of the preceding claims, **characterized in that** the fixation means are equipped with means for inserting the object to be secured into a location, for example, in a rack, said introduction means comprising preferably a spike parallel to the rear wall (150) of the object and capable of performing a circular movement relative to the rear wall (150), said spike being capable of being inserted into a guiding groove provided in one of the lateral walls of the location of the rack, so that the movement of the spike in the guiding groove causes the object to be secured to enter the location.

16. Device (100) according to the preceding claim, **characterized in that** a sensor is provided to detect a good positioning of the object to be secured in the location, the sensor being preferably a Hall effect sensor cooperating with a magnet placed in the pivotable fixation means, preferably in the vicinity of the spike.

17. Device (100) according to one of claims 1 to 16, **characterised in that** the locking means are equipped with a tab (130) equipped with a bearing surface dimensioned so as be capable of cooperating with the inner surface of the tightening means (120), as well as by a rod (131) that can serve as rotation axis (N) for the tightening means (120) and/or by an unlock button (133) that can be secured in particular to the other extremity of the rod (131) and/or by a spring (132).

## Patentansprüche

1. Vorrichtung (100) zur Befestigung eines Objekts auf einem Träger, welche das zu befestigende Objekt aufweist und mit Befestigungsmitteln ausgestattet ist, die eine Platte (110) umfassen, die an einer der Wände des zu befestigenden Objekts, vorzugsweise der Hinterwand, befestigt ist, sowie Klemmmittel (120), um die Befestigungsmittel an den Träger zu klemmen, wobei die Klemmmittel (120) bezogen auf die Platte (110) derart um eine Achse (N) schwenken können, dass sie gegen die Platte (110) geklappt oder senkrecht zur Platte (110) aufgerichtet werden können, wobei Verriegelungsmittel vorgesehen sind, um die Klemmmittel (120) in der Verwendungsposition senkrecht zur Platte (110) zu verriegeln, **dadurch gekennzeichnet, dass** die Verriegelungsmittel aus einem Zapfen (130) gebildet sind, der zwischen einer ersten eingefahrenen Position, in der er, wenn die Klemmmittel (120) in der aufgerichteten Position sind, durch eine Auflagefläche gegen die Innenseite (124) der Klemmmittel (120) platziert ist und diese in dieser Position blockiert, und einer zweiten entfernten Position versetzt werden kann, in der es den Klemmmitteln (120) möglich ist, frei in Richtung der Platte (110) zu schwenken, indem sie an dem entfernten Zapfen (130) vorbeigehen, so dass sich der Zapfen (130) in der geklappten Position der Klemmmittel (120) gegenüber einer oder gegen eine der Seiten (125) der Klemmmittel (120), die nicht die Unterseite (124) ist, befindet.

2. Vorrichtung (100) gemäß Anspruch 1, **dadurch gekennzeichnet, dass** sie mit einer Feder (132) versehen ist, die den Zapfen (130) in der eingefahrenen Position hält.

3. Vorrichtung (100) gemäß Anspruch 2, **dadurch gekennzeichnet, dass** der äußere Rand (125) der Klemmmittel (120) mit einer geneigten Fläche (126) ausgestattet ist, gegen die der Zapfen (130) beim Schwenken der Klemmmittel (120) zwischen deren geklappter und aufgerichteter Position gleiten kann, wobei der Zapfen (130) während dieser Schwenkbewegung um den äußeren Rand (125) der Klemmmittel (120) gegen die Kraft der Feder (132) herumgeht, bis er an dem äußeren Rand (125) vorbeigegangen ist und die Klemmmittel (120) in ihrer aufgerichteten Position blockiert.

4. Vorrichtung (100) gemäß einem der vorausgegangenen Ansprüche, **dadurch gekennzeichnet, dass** der Zapfen (130) mit einem Entriegelungsknopf (133) ausgestattet ist, auf den gedrückt werden kann oder der gezogen werden kann, um den Zapfen (130) in seine entfernte Position zu versetzen, gegebenenfalls gegen die Kraft der Feder (132).

5. Vorrichtung (100) gemäß einem der vorausgegangenen Ansprüche, **dadurch gekennzeichnet, dass** der Zapfen (130) auf der Rotationsachse (N) der Klemmmittel (120) platziert ist.

6. Vorrichtung (100) gemäß einem der Ansprüche 2 oder 3, **dadurch gekennzeichnet, dass** die Feder (132) eine Kompressionsfeder ist, die vorzugsweise im Innern der Rotationsachse (N) der Klemmmittel (120) platziert ist.

7. Vorrichtung (100) gemäß einem der vorausgegangenen Ansprüche, **dadurch gekennzeichnet, dass** der Zapfen (130) zwischen seinen beiden Endpositionen gemäß einer Translationsbewegung versetzt werden kann.

8. Vorrichtung (100) gemäß einem der vorausgegangenen Ansprüche, **dadurch gekennzeichnet, dass** die Klemmmittel (120) mit Betätigungsmitteln (122) ausgestattet sind, die derart auf den Klemmmitteln (120) angeordnet sind, dass sie von einer Seite der Befestigungsmittel bedient werden können.

9. Vorrichtung (100) gemäß einem der vorausgegangenen Ansprüche, **dadurch gekennzeichnet, dass** die Klemmmittel einen Spannkopf (120) aufweisen, der mit einer Spannbacke (121) und einer Klemmschraube (122) ausgestattet ist, die beide um eine Achse (N) schwenken können.

10. Vorrichtung (100) gemäß einem der vorausgegangenen Ansprüche, **dadurch gekennzeichnet, dass** die Platte (110) um 90° um eine Achse (P) schwenken kann, wodurch die Befestigung des Objekts auf einem vertikalen Träger oder auf einem horizontalen Träger in gleicher Weise ermöglicht wird.

11. Vorrichtung (100) gemäß einem der vorausgegangenen Ansprüche, **dadurch gekennzeichnet, dass** Arretiermittel vorgesehen sind, um die Schwenkbewegung der schwenkenden Befestigungsmittel (110, 120) zu begrenzen, vorzugsweise zwischen zwei Endpositionen, die um 90° zueinander gelegen sind, wobei die Arretiermittel vorzugsweise einen Zahn (111) zur Führung und zum Arretieren aufweisen, der mit der Platte (110) fest verbunden ist und mit einer Führungsnut (102), die in der Wand (150) des zu befestigenden Objekts ausgeführt ist, oder umgekehrt, zusammenwirkt.

12. Vorrichtung (100) gemäß einem der Ansprüche 10 oder 11, **dadurch gekennzeichnet, dass** die Führungsmittel vorgesehen sind, um die Schwenkbewegung der Klemmmittel (120) in die eine oder andere Verwendungsposition zu führen, wobei die Führungsmittel vorzugsweise eine Führungsrippe (127), die an den Klemmmitteln (120) ausgeführt ist, und mindestens eine Führungsrille (106), die in der Wand (150) des zu befestigenden Objekts gegenüber wenigstens einer der Verwendungspositionen ausgeführt ist und in die die Führungsrippe eindringen kann, wenn die Klemmmittel (120) um die Achse (N) in Richtung der Verwendungsposition schwenken, aufweist.

13. Vorrichtung (100) gemäß einem der Ansprüche 10 bis 12, **dadurch gekennzeichnet, dass** Blockiermittel vorgesehen sind, um die Platte (110) in wenigstens einer ihrer Verwendungspositionen zu blockieren, wobei die Blockiermittel vorzugsweise einen Index aufweisen, der auf den Klemmmitteln (120) platziert ist, der, wenn die Klemmmittel (120) in der Verwendungsposition aufgerichtet sind, in eine in der Wand des zu befestigenden Objekts ausgeführte Indexierungsnut eindringt.

14. Vorrichtung (100) gemäß einem der vorausgegangenen Ansprüche, **dadurch gekennzeichnet, dass** die schwenkenden Klemmmittel (120) eine Rille aufweisen, deren Abmessungen den gewöhnlichen Abmessungen der horizontalen Träger entsprechen, wobei die Rille vorzugsweise in der Spannbacke (121) der Klemmmittel (120) ausgeführt ist.

15. Vorrichtung (100) gemäß einem der vorausgegangenen Ansprüche, **dadurch gekennzeichnet, dass** die Befestigungsmittel mit Mitteln ausgestattet sind, um das zu befestigende Objekt an einer Stelle, zum Beispiel in einem Gestell, eindringen zu lassen, wobei die Einführmittel vorzugsweise einen Zahn aufweisen, der zur Hinterwand (150) des zu befestigenden Objekts parallel ist und bezogen auf die Hinterwand (150) eine Kreisbewegung beschreiben kann, wobei der Zahn in eine Führungsnut eindringen kann, die in einer Seitenwand der Stelle des Gestells ausgeführt ist, so dass durch die Bewegung des Zahns in der Führungsnut das Eintreten des zu befestigenden Objekts auf die Stelle hervorgerufen wird.

16. Vorrichtung (100) gemäß dem vorausgegangenen Anspruch, **dadurch gekennzeichnet, dass** ein Sensor vorgesehen ist, um die genaue Positionierung des zu befestigenden Objekts an der Stelle zu detektieren, wobei der Sensor vorzugsweise ein Hall-Sensor ist, der mit einem in den Befestigungsmitteln, vorzugsweise in der Nähe des Zahns, platzierten Magneten zusammenwirkt.

17. Vorrichtung gemäß einem der Ansprüche 1 bis 16, **dadurch gekennzeichnet, dass** die Verriegelungsmittel mit einem Zapfen (130) ausgestattet sind, der mit einer Auflagefläche ausgestattet ist, die so bemessen ist, dass sie mit der Innenfläche der Klemmmittel (120) zusammenwirkt, sowie mit einer Stange (131), die für die Klemmmittel (120) als Rotationsachse (N) dienen kann, und/oder mit einem Entriegelungsknopf (133), der insbesondere am anderen Ende der Stange (131) befestigt werden kann, und/oder mit einer Feder (132).
